(19) Europäisches Patentamt
European Patent Office
Office européen des brevets

(11) EP 4 751 704 A1

(12) **DEMANDE DE BREVET EUROPEEN**

(43) Date de publication:
**03.06.2026 Bulletin 2026/23**

(21) Numéro de dépôt: **25218373.6**

(22) Date de dépôt: **25.11.2025**

(51) Classification Internationale des Brevets (IPC):
***A61K 8/06*** *(2006.01)* ***A61K 8/26*** *(2006.01)*
***A61K 8/92*** *(2006.01)* ***A61P 17/10*** *(2006.01)*
***A61Q 19/00*** *(2006.01)* ***A61Q 19/08*** *(2006.01)*
***A61K 9/00*** *(2006.01)* ***A61K 47/44*** *(2017.01)*

(52) Classification Coopérative des Brevets (CPC):
**A61K 8/26; A61K 8/062; A61K 8/922; A61K 9/0014; A61K 47/44; A61P 17/10; A61Q 19/00; A61Q 19/08**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **28.11.2024 FR 2413152**

(71) Demandeur: **Ephyla**
**56190 Arzal (FR)**

(72) Inventeur: **BOURGETEAU, Vincent**
**56130 FEREL (FR)**

(74) Mandataire: **Eveillard, Sophie**
**Apropis**
**44, rue César**
**56100 Lorient (FR)**

(54) **COMPOSITION PHARMACEUTIQUE COMPRENANT UNE ÉMULSION DE PICKERING H/E CONTENANT UN PHYLLOSILICATE ORGANOMODIFIÉ NOTAMMENT POUR SON UTILISATION EN TANT QU AGENT ANTI-INFLAMMATOIRE, ET ANTI-ALLERGIE**

(57) Composition pharmaceutique comprenant une émulsion de Pickering H/E contenant une phase aqueuse, une phase huileuse végétale et un phyllosilicate organomodifié, notamment pour son utilisation pour augmenter la production de TAFA4, et éventuellement pour augmenter la production de sérotonine, pour son utilisation en tant qu'agent anti inflammatoire cutané et/ou en tant qu'agent anti-allergie cutanée.

EP 4 751 704 A1

## Description

La présente invention se rapporte au domaine médical et plus particulièrement à une composition pharmaceutique comprenant une émulsion de Pickering H/E particulière notamment pour son utilisation en tant qu'agent anti-inflammatoire et/ou anti-allergie.

La peau est la principale barrière de protection du corps humain à l'égard des agressions extérieures telles que la pollution atmosphérique, les variations climatiques, le rayonnement UV et également à l'égard des antigènes potentiellement nocifs.

La peau est constituée de trois couches : l'épiderme, le derme et l'hypoderme. L'épiderme, qui est la couche superficielle, est plus particulièrement concerné par les interactions avec le milieu extérieur. L'épiderme est recouvert par un film hydrolipidique et est composé de quatre couches : la couche cornée, la couche granuleuse, la couche épineuse et la couche basale. L'épiderme est normalement constitué de quatre types de cellules : les kératinocytes qui représentent 80% de l'ensemble de ses cellules, les 20% restant étant composés des mélanocytes, des cellules de Langerhans ou cellules immunocompétentes et des cellules de Merkel, ces trois groupes de cellules étant dispersées entre les kératinocytes.

Lorsqu'un antigène, ou plus généralement un agent pathogène, franchit la barrière que constitue la peau, une réponse inflammatoire est déclenchée par le système immunitaire de l'organisme.

Cette réponse se traduit par la production de cellules T, de cellules polynucléaires et de macrophages. Normalement cette réponse inflammatoire est contrôlée et s'arrête dès l'élimination de l'agent pathogène.

La protéine TAFA4 (ou FAM19A) est une neurokine de 12 kDa appartenant aux protéines de la famille TAFA. Cette protéine est impliquée dans divers processus de régulation du cycle cellulaire comme la modulation de la réponse immunitaire : phagocytose, libération d'espèces réactives de l'oxygène (ROS) ou chimiotactisme des macrophages. TAFA4 est principalement exprimée dans les nocicepteurs, il s'agit d'un ligand des mécanorécepteurs à bas seuil C ou C-LTMRs (« C-low-treshold » en langue anglaise). Cette neurokine est un marqueur spécifique des C-LTMRs et est impliquée dans la perception de la douleur dans la moelle épinière.

Les protéines TAFA représentent une nouvelle classe de ligands de type chimiokine qui se révèlent être des protéines sécrétoires dérivées des neurones ou neurokines, elles sont notamment impliquées dans la régulation des réponses immunitaires au sein du système nerveux central. En tant que protéine sécrétée, la TAFA4 se lie au récepteur du peptide de formol 1 (FPR1), un récepteur couplé à la protéine G, à la surface de la cellule, et agit comme médiateur de la chimio-attraction des macrophages, la phagocytose et le profil inflammatoire des macrophages.

Les publications (1) et (2) attestent du rôle de TAFA4 dans la régulation des désordres inflammatoires cutanés et la régénération des tissus, et la demande de brevet WO 2020/064907 divulgue une méthode de réduction de l'inflammation de la peau comprenant l'administration à un sujet d'une quantité active d'un polypeptide de type TAFA4 ou d'une molécule d'acide nucléique codant TAFA4. La publication (4) atteste du rôle de TAFA4 dans l'immunothérapie specifique aux allergènes.

Il demeure toujours un besoin de nouvelles compositions pharmaceutiques aptes à contrôler et réduire l'inflammation cutanée, ainsi que les allergies cutanées.

Par ailleurs les disques de Merkel sont des récepteurs superficiels situés à la base de l'épiderme et composés de la terminaison d'une ramification (en forme de disque) d'une fibre myélinisée apposée à une cellule de Merkel avec laquelle elle établit des contacts synaptiques. Les zones riches en disques de Merkel peuvent former des dômes tactiles qui répondent à des pressions localisées, la réponse au stimulus étant phasico-tonique à adaptation lente.

Récemment, des recherches sur les cellules de Merkel se sont focalisées sur leur fonction dans la mécanosensation, en particulier le toucher léger (« light -touch » en langue anglaise), en raison de leur rôle primordial dans les tâches sensorielles et les interactions sociales.

En particulier, il a été été mis en évidence que les cellules de Merkel utilisent la sérotonine pour transmettre les stimulations tactiles aux terminaisons des nerfs A$\beta$-afférents avec lesquels elles sont en contact et que les stimuli tactiles activent des canneaux Piezo 2 pour transduire les stimuli mécaniques en stimuli électriques conduisant à générer des impulsions sur les nerfs A$\beta$-afférents (3).

Ces recherches ont mis en évidence l'importance de la sérotonine dans la neurotransmission des informations provenant des cellules cutanées de l'épiderme.

La sérotonine est un neuro-médiateur qui se transmet aussi bien à la microfibrille nerveuse sous-jacente aux groupes de cellules de Merkel (au niveau de la couche basale de l'épiderme), qu'aux cellules de Langerhans au niveau de l'épiderme. Les cellules de Langerhans sont bio stimulées positivement par le médiateur « sérotonine ». Les cellules de Langerhans sont la première ligne du système immunitaire, en stimulant ces cellules, le système immunitaire se renforce.

La sérotonine produite dans les cellules de Merkel présente des propriétés de neurotransmetteurs et est capable de transmettre au cerveau un message positif provenant de la peau (exemple « sensation de caresse ») et d'exercer un effet bénéfique (de type neuro-protecteur) sur les neurones sensoriels présent dans la peau.

De manière inattendue et avantageuse, l'inventeur a observé qu'une composition comprenant une émulsion de Pickering H/E particulière permet à la fois d'augmenter la sécretion de serotonine et aussi la sécretion des TAFA4.

## Résumé de l'invention

L'inventeur a mis en œuvre une nouvelle composition pharmaceutique comprenant une émulsion de Pickering particulière présentant notamment des propriétés anti-inflammatoire et/ou anti-allergie.

Selon un premier aspect, la présente invention vise une composition pharmaceutique comprenant une émulsion de Pickering H/E contenant une phase aqueuse, une phase huileuse végétale et un phyllosilicate organomodifié.

Selon un deuxième aspect, la présente invention vise ladite composition pharmaceutique pour son utilisation pour augmenter la production de TAFA4.

Selon un troisième aspect, la présente invention vise ladite composition pharmaceutique pour son utilisation pour augmenter la production de sérotonine.

Selon un quatrième aspect, la présente invention vise ladite composition pharmaceutique pour son utilisation en tant qu'agent anti inflammatoire cutané et/ou en tant qu'agent anti-allergie cutanée.

Selon un cinquième aspect, la présente invention vise ladite composition pharmaceutique pour son utilisation en tant qu'agent anti inflammatoire cutané pour la prévention et/ou le traitement d'une maladie inflammatoire de la peau de préférence choisie dans le groupe formé par l'acné, la rosacée, la folliculite, la dermite périorale, le photovieillissement, le vieillissement cutané, le psoriasis, l'ichtyose, les plaies chroniques, les escarres, la kératose pilaire, , les kystes sébacés, les dermatoses inflammatoires, l'hyperpigmentation post-inflammatoire, la xérose, le prurit, le lichen plan, le prurigo nodulaire, l'eczéma, la fièvre miliaire, la sclérodermie, la dermatite atopique, la dermopathie fibrosante néphrogénique, la connectivité mixte, le scléromyxoedème, le scléroedème, la chéloïde, la sclérodactylie, la fasciite à éosinophiles , la photodermatose, les ulcères de stase veineuse ou d'ulcères du pied diabétique, la fibrose cutanée.

Un autre avantage de la composition selon l'invention est qu'elle confère un effet apaisant aux peaux qui ont subit un stress, plus généralement elle présente une action eudermique c'est-à-dire qu'elle crée une sensation de bien être lorsqu'elle est appliquée sur la peau.

Les compositions selon l'invention sont en outre stables, non irritantes, non toxiques, non allergisantes pour la peau.

D'autres aspects, avantages, propriétés de la présente invention sont présentés dans la description et les exemples qui suivent.

## Description détaillée

### Définitions

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Sauf indications contraires, un intervalle défini comme étant « compris entre la valeur A et la valeur B » inclut les valeurs A et B et est donc équivalent à un intervalle « allant de la valeur A à la valeur B » , l'expression « au moins » comprend la valeur énoncée après, par exemple, «au moins 5 % » doit être compris comme comprenant également « 5 % », l'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de ± 5 %.

Par « peau » on entend l'épiderme du visage ou du corps ou du cuir chevelu.

Sans vouloir être lié à une théorie quelconque, il apparait que l'application sur la peau de la composition comprenant une émulsion de Pickering particulière permet la production de sérotonine également appelée 5-hydroxytryptamine (5-HT) au niveau de la peau *via* la stimulation des cellules de Merkel. La sérotonine est, en effet, un neurotransmetteur connu pour faciliter la communication des neurones du système nerveux central. En réponse à ce message positif les neurones émettent de la TAFA4 : ce médiateur qui est en attente de maturation dans les neurones voit sa maturation activée sous l'action de la sérotonine avant d'être libéré. Or, la libération de TAFA4 conduit à réduire l'inflammation cutanée.

En outre, la composition pharmaceutique selon l'invention, grâce à sa structure galénique particulière procure à la peau un effet biomimétique du réconfort que procure une légère caresse ou encore l'action de souffler sur une égratignure, cet effet peut encore être nommé d'« effet caresse ».

Cet effet « caresse » est particulièrement présent lorsque la composition est appliquée par pulvérisation. Les fines gouttelettes déposées restent sur la surface de la peau imitant ainsi les cornéocytes. La légère pression exercée sur la peau permet de stimuler les mécanorécepteurs et de libérer des neuropeptides qui envoient au cerveau des signaux similaires à ceux d'une caresse légère et agréable. Ce signal est apte à endormir tout message d'inconfort envoyé au cerveau pour le remplacer par un message de bien être.

### Emulsions de Pickering

Les émulsions de Pickering sont des émulsions stabilisées par des particules solides. Lors de la préparation de l'émulsion, lesdites particules solides se positionnent à l'interface entre la phase aqueuse et la phase huileuse.

Les émulsions de Pickering utilisées selon la présente invention sont des émulsions huile dans eau, c'est-à-dire H/E, stabilisées par une argile particulière qui est un phyllosilicate naturel modifié.

Avantageusement, la phase aqueuse de l'émulsion de Pickering est présente en une quantité allant de 43% à 93%, de préférence 55% à 70% et de manière préférée 58% à 65% en poids par rapport au poids total de la composition.

Selon un mode de réalisation préféré, le phyllosilicate naturel modifié comprend un phyllosilicate choisi dans le groupe constitué par les vermiculites et les smectites. De préférence, le phyllosilicate peut être choisi dans le groupe constitué par les montmorillonites, bentonites, nontronites, beidellites, volkonskoites, hectorites, saponites, sauconites, sobockites, stevensites, svinfordites, de préférence ce sont des phyllosilicates de sodium, de potassium, de calcium, ou de leurs mélanges. De manière plus préférée, le phyllosilicate est choisi dans le groupe constitué par l'hectorite, la montmorillonite, la bentonite ou leurs mélanges.

Selon un mode de réalisation préféré, le phyllosilicate naturel modifié comprend un composé organique choisi dans le groupe constitué par la gomme xanthane, la gomme guar, la gomme de Tara ou de Caroube, la gomme d'Acacia, le carraghénane, l'alginate, le chitosane, la pectine, l'acide citrique, l'acide tartrique, l'acide oxalique, l'acide succinique, l'acide malique, l'acide acétique, l'acide lactique, l'acide propionique, l'acide salicylique, les glycosaminoglycanes. Avantageusement, le phyllosilicate naturel modifié comprend une bentonite modifiée par la gomme de xanthane et l'acide citrique.

Selon un autre mode de réalisation, le phyllosilicate naturel modifié comprend un composé organique choisi parmi tout composé organique connu dans l'art des émulsions de Pickering, tels que ceux divulgués dans la demande de brevet français n° FR 2 976 503.

Avantageusement, le phyllosilicate modifié est présent en une quantité allant de 2% à 20%, de préférence 2,5% à 10% et de manière préférée 3,5% à 8% en poids par rapport au poids total de la composition.

Selon un mode de réalisation préféré, la phase huileuse vegétale de l'émulsion de Pickering comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/caprique.

Avantageusement la composition utilisée dans la présente invention ne comprend pas d'extrait d'huile de chanvre.

Avantageusement, la phase huileuse de l'émulsion de Pickering est présente en une quantité allant de 5% à 40%, de préférence 10% à 30% et de manière préférée 15% à 25% en poids par rapport au poids total de la composition.

### Composition

La composition pharmaceutique selon l'invention est de préférence une composition vaporisable (« sprayable » en langue anglaise) en fines gouttelettes.

La composition pharmaceutique selon l'invention peut également être une émulsion de type crème ou pommade H/E dont la viscosité n'autorise pas la vaporisation, mais l'application topique directe. Dans ce cas, les microcapsules sont dispersées à la surface de la peau par massage du bout des doigts.

De préférence, la composition utilisable selon l'invention comprend de 43 à 93 % en poids d'eau par rapport au poids total de la composition.

Avantageusement la composition selon la présente invention comprend, dans un milieu acceptable, au moins un agent additionnel choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

De manière particulière la composition pharmaceutique selon l'invention comprend avantageusement au moins une amyrine. L'amyrine est avantageusement choisie dans le groupe formé par l'$\alpha$ amyrine (à 100% ou viminalol), la $\beta$ amyrine (à 100%), un mélange d'$\alpha$ et $\beta$ amyrines contenant de 20% à 80% d'$\alpha$ amyrine et de 20% à 80% de $\beta$ amyrine de préférence d'un mélange d'$\alpha$ et $\beta$ amyrines contenant de 20% à 80% d'$\alpha$ amyrine et de 20% à 80% de $\beta$ amyrine.

Avantageusement la composition selon la présente invention comprend de 0,001 à 20 % en poids d'au moins un agent additionnel par rapport au poids total de la composition.

Bien entendu, l'homme du métier veillera à choisir ces agents additionnels de manière à ne pas altérer les propriétés de la composition utilisable selon l'invention, notamment de manière à ne pas altérer les propriétés conférant à cette composition son caractère vaporisable.

De manière avantageuse, le procédé de préparation de la composition selon l'invention comprend au moins les étapes suivantes dans cet ordre : préparer la phase aqueuse ;
ajouter le phyllosilicate organomodifié à la phase aqueuse et mélanger ; ajouter la phase huileuse végétale au mélange obtenu et obtenir une émulsion de Pickering H/E.

**Utilisations**

La composition pharmaceutique selon l'invention est avantageusement destinée à être appliquée sur la peau par pulvérisation, vaporisation.

Les exemples qui suivent visent à illustrer l'invention sans en limiter la portée.

**Exemples**

**A-Compositions**

Dans le tableau 1 sont répertoriés les produits utilisés pour préparer la composition **A** utilisable selon l'invention.

[Tableau 1]

| Phase | Nom commercial | % | INCI |
|---|---|---|---|
| A | Eau | Qsp | Aqua |
| A | Frametime CXG commercialisé par Ephyla | 2,50 | Bentonite & xanthan gum & Sodium stearoyl glutamate & citric acid |
| B | Huile de tournesol raffinée commercialisée par CAUVIN | 8,00 | Helianthus annuus seed oil |
| C | Georgard Ultra commercialisé par Lonza | 1,00 | Gluconolactone & Sodium benzoate & Calcium gluconate |
| C | Benzoate de Sodium | 0,30 | Sodium benzoate |
| Total | | 100 | |

Préparation de la composition **A**

Les constituants de la phase A ont été mélangés à 20°C avec un mixeur mécanique à couteaux ou un rotor stator à une vitesse de 4000 rpm durant 10 minutes de sorte à mettre en œuvre un cisaillement et une dispersion suffisante pour obtenir l'homogénéisation de la phase.

La phase B a ensuite été incorporée, toujours à 20°C, sur le même modèle de cisaillement progressivement durant 10 minutes.

L'émulsion de pickering a ainsi été réalisée. La température du mélange, sous cisaillement, a ensuite été augmentée pour atteindre 50°C de sorte à pouvoir solubiliser le conservateur.

La phase C (conservateurs) a ensuite été introduite à cette température de 50°C sous une agitation plus modérée de 2000 rpm.

Le produit est refroidi à température ambiante.

Enfin, le pH a été ajusté à 4,9.

La galénique de la composition **A** obtenue comprend des fines gouttelettes d'huile enrobées de plaquettes de bentonite. Ces plaquettes minérales sont stables à l'interface huile/eau, elles sont issues de l'exfoliation de la bentonite (Frametime CXG) sous l'action du cisaillement mécanique et forment des microcapsules d'huile. La dispersion des microcapsules est stable lorsque le produit est au repos même si sa viscosité est faible.
La taille des microcapsules est de l'ordre de 5 à $15.10^{-6}$ m de diamètre, ce qui permet à cette galénique de passer à travers les buses de vaporisation de type standard en cosmétique.
Cette composition **A** a ainsi été vaporisée sur la peau d'un bras présentant des signes d'inflammation. Au bout de 10 heures après application, on a noté une nette diminution de l'état inflammatoire : disparition de la sensation de chaleur et des signes visuels d'une inflammation cutanée.
Dans le tableau 2 sont répertoriés les produits utilisés pour préparer la composition **B** utilisable selon l'invention.

[Tableau 2]

| Phase | Nom commercial | % | INCI |
|---|---|---|---|
| A | Eau | 73,70 | Aqua |
| B | Georgard Ultra commercialisé par Lonza | 1,00 | Gluconolactone & Sodium benzoate & Calcium gluconate |
| B | Benzoate de Sodium | 0,30 | Sodium benzoate |
| C | Frametime CX commercialisé par Ephyla | 4,50 | Bentonite & xanthan gum & citric acid |
| C | Gommme de xanthane FF commercialisée par Jungbunzlauer | 0,50 | xanthan gum |
| D | Trigycéride d'acides caprique /caprylique | 20,00 | Caprylic/capric triglyceride |
| Total | | 100 | |

Préparation de la composition **B** :
Les phases A et B ont été mélangées pour former une phase aqueuse puis la phase C a été ajoutée et le tout a été mélangé. Enfin la phase huileuse D a été ajoutée et le tout a été mélangé. Une fois l'émulsion de Pickering obtenue, le pH a été ajusté à 5,00.
La galénique de la composition **B** obtenue comprend des fines gouttelettes d'huile enrobées de plaquettes de bentonite. Ces plaquettes minérales sont stables à l'interface huile/eau, elles sont issues de l'exfoliation de la bentonite (Frametime CXG) sous l'action du cisaillement mécanique et forment des microcapsules d'huile. La dispersion des microcapsules est stable lorsque le produit est au repos, sa viscosité correspond à celle d'une crème qui tient dans un pot de crème standard.
Cette composition **B** a été étalée manuellement sur la peau égratignée d'un genou. La crème étalée procure un effet caresse durable, réconfortant et apporte une sensation de bien-être.
Ce type de galénique « microencapsulée » permet de déposer ou brumiser une couche de microcapsules contenant des molécules lipidiques de soin de la peau. En séchant, les microcapsules libèrent progressivement les molécules lipidiques, ce qui permet d'apporter un effet hydratant et des substances anti-inflammatoires avec une grande rémanence, ainsi que d'améliorer l'hydratation de la peau eau fur et à mesure de la libération de microcapsules.
Ainsi la galénique participe à équilibrer la peau en surface en paliant spécifiquement à la sécherresse cutanée des zones les plus sèches et en n'ajoutant pas de corps gras sur les zones grasses.

**B** - **Mesure de l'augmentation de la production de sérotonine et l'augmentation de la production de TAFA4**

*Compositions testées*

La composition **A** est utilisée comme composition conforme à l'invention.
A titre de comparaison, ont été utilisés :

du sérum physiologique c'est-à-dire une composition de NaCl à 0.9%, à titre de témoin basal (TB) et
de l'isothiocyanate de1-naphtyle à la concentration de 0,25% (V/V) à titre de témoin positif (TP).

*Principe de l'étude*

Pour cette étude des explants cutanés humains frais normaux avec cellules de Merkel sont incubés pendant 20 heures à 32°C pour acclimatation.

Puis les échantillons d'explants à tester -en triplicat- sont traités par pulvérisation de la composition A utilisée conformément à la l'invention ou par pulvérisation des compositions témoins TB et TP et replacés en incubation à 32°C.

Au bout de 3 heures, une première série d'échantilllons d'explants traités a été récupérée et il a été procédé à la dissection et à la mise en préparation des explants traités : placement dans l'azote liquide pendant 3 minutes, puis dans 10 mL de PBS 1X glacé puis dans un bain d'ultrasons pendant 45 minutes de sorte à obtenir un lysat cellulaire.

Après ce traitement réalisé pour chaque implant, 2mL de lysat cellulaire de chaque d'échantilllon d'explant de cette première série ont été prélevés et 1% d'agent stabilisant y a été ajouté pour les conserver à -20°C jusqu'à la réalisation des dosages Elisa.

Les explants traités pendant 3 heures au moyen des compositions A, TB et TP sont respectivement nommés A3, TB3 et TP3.

Au bout de 8 heures, une deuxiéme série d'échantilllons d'explants traités a été récupérée et traitée comme ci-dessus. Puis 2mL de lysat cellulaire de chaque d'échantilllon d'explant de cette deuxième série ont été prélevés et 1% d'agent stabilisant y a été ajouté pour les conserver à -20°C jusqu'à la réalisation des dosages Elisa.

Les explants traités pendant 8 heures aux compositions A, TB et TP sont respectivement nommés A8, TB8 et TP8.

Au moment de la réalisation des dosages Elisa, les échantilllons d'explants traités ont été soumis à un nouveau bain à ultrasons pendant 35 minutes puis, après homogénéisation, les surnageants sont récupérés en vue de la réalisation des tests Elisa.

<u>B1 Mesure de l'augmentation de la production de sérotonine</u>

Les cellules de Merkel de la peau libérent des neurotransmetteurs et en particulier de la serotonine de sorte à transmettre au cerveau l'information d'un toucher « caresse » *via* la fibrille nerveuse sous-jacente. C'est ainsi que les mecanorécepteurs (cellules de Merkel ou corpuscule de Merkel) assurent notre sens du toucher notamment sur les sensations positives ; il s'agit de transcrire un effet mécanique, à savoir le toucher « caresse », en message chimique, à savoir la sérotonine, un neuromédiateur dont le cerveau assimile au bien-être.

Cette étude est basée sur la capacité de la composition conforme à l'invention **A** à augmenter la production de sérotonine.

L'effet recherché est un effet stimulateur sur la production de sérotonine qui se traduit par un effet de réconfort, une sensation de bien-être, un effet caresse sur l'épiderme qui est en contact avec le milieu extérieur.

Système d'essai Elisa - sérotonine

Les solutions de standard et de contrôle ont été préparées conformément aux indications du kit Elisa - sérotonine (référence commerciale du kit : Serotonin Research ELISA ® commercialisé par Immusmol).

Le kit fournit un test pratique pour le dosage de la concentration en sérotonine des milieux comprenant les surnageants d'explants cutanés préparés permettant ainsi d'évaluer l'effet modulateur de la composition topique à tester.

Ce kit met en œuvre une méthode d'immunodosage enzymatique ultrasensible pour la détermination quantitative de la sérotonine. La sérotonine est acylée, puis détectée par les antigènes qui sont liés à la phase solide de la plaque de microtitrage. Les normes, contrôles et échantillons acylés et les analytes liés à la phase solide sont en concurrence pour un nombre fixe de sites de liaison d'anticorps. Une fois le système en équilibre, l'antigène libre et les complexes antigènes libres-anticorps sont éliminés par lavage. L'anticorps lié à la phase solide est détecté par un conjugué IgG-peroxydase anti-lapin en utilisant TMB ((3,3',5,5'-Tetramethylbenzidine) comme substrat. Il s'agit donc d'un test colorimétrique dont la réaction colorée est révélée à 450 nm. La quantification des échantillons inconnus est obtenue en comparant leur absorbance avec une courbe standard préparée avec des concentrations standard connues.

*Résultats*

<u>Gamme étalon</u>

Pour cette étude une gamme d'étalonnage en sérotonine allant de 0,0 à 2,5 $10^{-9}$ g/mL (ng/mL) a été réalisée. Les absorbances de la gamme étalon en sérotonine ( densité optique(DO)) à 450 nm sont présentés dans le tableau 3.

[Tableau 3]

| $DO_{450nm}$ | | | | | | |
|---|---|---|---|---|---|---|
| | Blanc | **Standards** = Gamme sérotonine ($10^{-9}$ g/mL ) | | | | |
| | S0 | S1 = 0,000 | S2 = 0,015 | S3 = 0,050 | S4 = 0,150 | S5 = 0,250 |
| Replicat 1 | 12,545 | 11,633 | 11,095 | 9,839 | 6,903 | 4,309 |
| Replicat 2 | 12,555 | 11,698 | 10,982 | 9,679 | 7,263 | 3,895 |
| Replicat 3 | 13,053 | 12,929 | 9,643 | 9,074 | 6,760 | 4,182 |
| Moyenne | 12,718 | 12,087 | 10,573 | 9,531 | 6,975 | 4,129 |
| *Ecart type* | *0,290* | *0,730* | *0,807* | *0,403* | *0,259* | *0,212* |

L'équation de la courbe d'étalonnage est :

$$y = 28{,}673x^2 - 25{,}137x + 12{,}393$$

le coefficient de détermination est : $R^2 = 0{,}9719$

Mesure des échantillons

Les résultats d'absorbance (DO) à 450 nm avec écarts types de la composition conforme A et des témoins TB et TP à 3 heures et 8 heures sont présentés respectivement dans le tableau 4, ci-dessous. Le pourcentage (%) d'augmentation par rapport au témoin basal correspondant.

[Tableau 4]

| DO 450 nm de la composition A et des témoins et ecart type | | | | | | |
|---|---|---|---|---|---|---|
| | TB3 | TP3 | A3 | TB8 | TP8 | A8 |
| DO 450nm | 0,204 | 0,340 | 0,246 | 0,203 | 0,254 | 0,246 |
| Ecart type | 0,020 | 0,031 | 0,028 | 0,026 | 0,060 | 0,025 |
| % d'augmen tation par rapport au témoin basal | 0 | 66,67 | 20,58 | 0 | 25,12 | 21,18 |

Dans les conditions de l'étude, la cinétique de réponse positive par rapport au temps de mise en contact avec les explants de peau montre que le pic de stimulation attendu avec le témoin positif (l'isothiocyanate de1-naphtyle) s'observe après 3 heures de contact avec + 66,67% de sérotonine produite par rapport au témoin explant de peau sans produit à tester. Cette réponse positive au témoin positif valide l'expérience. Après 8 heures de contact l'effet positif du témoin positif diminue très significativement, l'effet est décroissant entre 3 heures de contact et 8 heures de contact.

Avec la composition A selon l'invention, l'augmentation de la production de serotonine, par rapport au témoin basal TB3 est de 20,58% au bout de 3 heures (A3), et reste identique au bout de 8 heures (21,18% au bout de 8h). L'effet positif de la composition A selon l'invention est donc stable dans le temps ce qui témoigne d'une rémanence sur cet effet positif.

Conclusion :

La composition selon l'invention **A** appliquée sur les explants exposés stimule la production de sérotonine de plus de 20% par rapport au témoin basal après 3 heures de mise en contact avec l'explant, cette production se maintient à + 21% par rapport au témoin basal après 8 heures de mise en contact (A8).

Il est rappelé que dans la peau les cellules de Merkel sont les mécanorécepteurs en première ligne du sens du toucher, ainsi que les principales cellules capables de produire de la sérotonine en quantité significative. La sérotonine est un neuro-médiateur de la sensation de caresse qui par la fibrille nerveuse sous-jacente aux cellules de Merkel permet d'informer le cerveau d'une sensation agréable est positive.

Ainsi, la composition selon l'invention A par augmentation de la production du neuro-médiateur sérotonine informe le cerveau d'une modulation positive de type caresse ou effet sensoriel positif.

B2 -Mesure de l'augmentation de la production de TAFA4

Les protéines TAFA sont des protéines sécrétoires dérivées des neurones ou neurokines, elles sont notamment impliquées dans la régulation des réponses immunitaires au sein du système nerveux central. En tant que protéine sécrétée, la TAFA4 se lie au récepteur du peptide de formol à la surface de la cellule, et agit comme médiateur de la chimio-attraction des macrophages, la phagocytose et le profil inflammatoire des macrophages.

Cette étude est basée sur la capacité de la composition conforme à l'invention **A** à augmenter la production de TAFA4.

L'effet recherché est un effet stimulateur sur la production de TAFA4 qui se traduit par un effet anti inflammatoire.

Pour cette étude, le kit ELISA TAFA-4 (Human Chemokine-Like Protein TAFA-4) fournit un test pratique pour le dosage de TAFA-4 et l'évaluation de la biostimulation de la réduction de l'inflammation cutanée par cette protéine.

Ce kit utilise des anticorps monoclonaux spécifiques de TAFA-4. L'interaction entre TAFA-4 et les anticorps est détectée par l'ajout d'un substrat conduisant à la production de couleur. L'intensité de la couleur est mesurée par spectrophotométrie à 450 nm et est proportionnelle à la concentration de TAFA-4. Une courbe d'étalonnage est utilisée pour déterminer la concentration de TAFA-4 dans les échantillons.

Les solutions de standard et de contrôle ont été préparées conformément aux indications du kit Elisa - TAFA4 (référence commerciale du kit : Human chemokine-Like Protein TAFA-4 commercialisé par Abbexa).

Le kit fournit un test pratique pour le dosage de la concentration en TAFA4 des milieux comprenant les surnageants d'explants cutanés préparés permettant ainsi d'évaluer l'effet modulateur de la composition topique à tester.

Ce kit est basé sur la technologie de dosage immuno-enzymatique sandwich. Un anticorps est pré-enduit sur une plaque à 96 puits. Des étalons, des échantillons de test et un réactif conjugué à la biotine sont ajoutés aux puits et incubés. Le réactif conjugué à la HRP (*Horseradish Peroxidase*) est ensuite ajouté et la plaque entière est incubée. Les conjugués non liés sont éliminés à l'aide d'un tampon de lavage à chaque étape. Le substrat TMB (le 3,3',5,5'-tétraméthylbenzidine ou TMB est un substrat chromogène utilisé en immunohistochimie et comme révélateur de la peroxydase dans la technique ELISA) est utilisé pour quantifier la réaction enzymatique HRP. Une fois le substrat TMB ajouté, seuls les puits contenant suffisamment de TAFA4 produiront un produit de couleur bleue, qui vire ensuite au jaune après l'ajout de la solution d'arrêt acide. L'intensité de la couleur jaune est proportionnelle à la quantité de TAFA4 liée sur la plaque. La densité optique (DO) est mesurée par spectrophotométrie à 450 nm dans un lecteur de microplaques, à partir duquel la concentration de TAFA4 peut être calculée.

*Résultats*

Gamme étalon

Pour cette étude une gamme d'étalonnage en TAFA4 allant de 0,0 à 10,00 $10^{-9}$ g/mL (ng/mL) a été réalisée. Les résultats : DO 450 nm (densité optique à 450nm) et courbe d'étalonnage sont présentés dans le tableau 5.

[Tableau 5]

| | $DO_{450nm}$ | | | | | | | |
|---|---|---|---|---|---|---|---|---|
| | Blanc S0 | **Standards =** Gamme TAFA4 ($10^{-9}$ g/mL ) | | | | | | |
| | | S1 = 0,16 | S2 = 0,31 | S3 = 0,63 | S4 = 1,25 | S5 = 2,50 | S6 = 5,00 | S7 = 10,00 |
| Repl. 1 | 0,156 | 0,251 | 0,314 | 0,471 | 0,791 | 1,561 | 2,709 | 4,176 |
| Repl.2 | 0,210 | 0,260 | 0,304 | 0,456 | 0,841 | 1,522 | 2,546 | 4,225 |
| Repl.3 | 0,227 | 0,230 | 0,307 | 0,422 | 0,872 | 1,462 | 2,500 | 4,094 |
| Moyen | 0,198 | 0,247 | 0,308 | 0,450 | 0,835 | 1,515 | 2,585 | 4,165 |
| Ecart type | 0,037 | 0,015 | 0,005 | 0,025 | 0,041 | 0,050 | 0,110 | 0,066 |

L'équation de la courbe d'étalonnage est :

$$y = 0{,}4193x$$

le coefficient de détermination est : $R^2 = 0,9906$

Système d'essai :

Pour cette étude, des disques de peau humaine fraîche de pleine épaisseur sont exposés au sérum physiologique avant le début de la période de contact. Ensuite, les disques de peau sont incubés pendant 15 à 20 heures à 32 ± 1 °C pour l'acclimatation. À la fin de la période d'acclimatation, des échantillons pour tester ou un contrôle basal (sérum physiologique) ou un contrôle positif (fourni par EPHYLA) sont pulvérisés sur les disques de peau avant une incubation de 8 heures à 32 ± 1 °C. Après le temps d'incubation, les disques de peau sont traités pour la production d'homogénats de tissu en utilisant du PBS 1X. Tous les échantillons sont conservés à -80 °C jusqu'au test TAFA4.

Mesure des échantillons

Les résultats d'absorbance (DO) à 450 nm avec écarts types de la composition conforme A et des témoins TB et TP à 8 heures sont présentés respectivement dans le tableau 6, ci-dessous. Le facteur (multiplicatif) d'augmentation par rapport au témoin basal correspondant

[Tableau 6]

| DO 450 nm de la composition A et des témoins et ecart type | | | | |
|---|---|---|---|---|
| | TB3' | A3' | TB8' | A8' |
| DO 450nm | 0,025 | 0,249 | 0,001 | 0,198 |
| Ecart type | 0,003 | 0,026 | 0,002 | 0,021 |
| Facteur (multiplicatif) d'augmentation par rapport au témoin basal | 0 | 9,96 | 0 | 198 |

Dans les conditions de l'étude, le taux basal est toujours très bas (TB3' et TB8'). Lorsque la composition A selon l'invention est appliquée, le taux d'augmentation de la sécrétion en TAFA4 augmente dès 3H après la mise en contact avec la peau (A3'), ce taux est toujours très élévé 8H après la mise en contact avec la peau (A8'). Cette réponse positive valide l'expérience et montre, qu'en réponse au message positif généré par la sérotonine, le neurone émet de la TAFA4 : ce mediateur qui est en attente de maturation dans le neurone voit sa maturation activée sous l'action de la sérotonine avant d'être libéré au bout d'une période comprise entre 3 h et 8h.

L'expérience a été répétée pour confirmation. Les résultats d'absorbance (DO) à 450 nm avec écarts types de la composition conforme A et du témoin basal TB à 8 heures sont présentés dans le tableau 7, ci-dessous, ainsi que le pourcentage (%) d'augmentation par rapport au témoin basal.

[Tableau 7]

| DO 450 nm de la composition A et des témoins et ecart type | | |
|---|---|---|
| | TB8" | A8" |
| DO 450nm | 0,143 | 0,312 |
| Ecart type | 0,008 | 0,018 |
| % d'augmentation par rapport au témoin basal | 0 | 118.2 |

Conclusion :

Le taux basal de TAFA4 est significatif avec un écart type très faible (ce taux est donc robuste et bien représentatif). Lorsque la composition A selon l'invention est appliquée, le taux de sécrétion de TAFA4 augmente significativement 8H après la mise en contact (A8" ci-dessus), ce résultat s'appuie sur un écart type très faible, il est donc robuste et bien représentatif.

La composition A selon l'invention permet donc d'accroître de plus 118% la quantité de TAFA4 dans la peau, cette augmentation est le fruit de la sécrétion de TAFA4 par les microfibrilles nerveuses. A l'instar d'une caresse sur la peau, la composition A selon l'invention stimule les mécanorécepteurs notamment ceux qui permettent la production de neuromédiateurs de type sérotonine. Les fibres nerveuses sensorielles sont stimulées positivement et sécrètent en récompense la TAFA4. Ce qui est surprenant c'est que les fibres nerveuses sont coupées dans un explant, néanmoins leurs terminaisons contiennent le métabolisme permettant la sécrétion de TAFA4, molécule induite dans sa production par le cerveau.

Ainsi, la composition selon l'invention A par augmentation de la production de TAFA4 conduit à réduire l'inflammation cutanée.

## BIBLIOGRAPHIE

1. Molecular Structure, Expression and Role of TAFA4 and its Receptor FPR1 in the Spinal Cord. Zhu S, Hu X, Bennett S, Mai Y and Xu J. Frontiers in Cell and Developmental Biology (2022) 10:911414.doi: 10.3389/fcell.2022.911414 B

2. Sensory neuron-derived TAFA4 promotes macrophage tissue repair functions Guillaume Hoeffel, Guilhaume Debroas, Anais Roger, Rafaëlle Rossignol, Jordi Gouilly, et al , Nature 2021, 594 (7861), pp.94-99 10.1038/s41586-021-03563-7 hal-03419356

3. Effects on tactile transmission by serotonin transporter inhibitors at Merkel discs of mouse whisker hair follicles, Chang and G Gu, Molecular Pain vol. 16 : 1-9, 20 may 2020

4. TAFA4-IL-10 axis potentiate immunotherapy for airway allergy by induction of specific regulatory T cells, Qiu et al. npj Vaccines (2022) 7:133 *;*


## Revendications

1. Composition pharmaceutique comprenant une émulsion de Pickering H/E contenant une phase aqueuse, une phase huileuse végétale et un phyllosilicate organomodifié, pour son utilisation pour augmenter la production de TAFA4.

2. Composition pharmaceutique selon la revendication 1 dans laquelle le phyllosilicate organomodifié est présent en une quantité allant de 2% à 20%, de préférence 2,5% à 10% et de manière préférée 3,5% à 8% en poids par rapport au poids total de la composition.

3. Composition pharmaceutique selon l'une quelconque des revendications 1 ou 2 dans laquelle la phase aqueuse de l'émulsion de Pickering est présente en une quantité allant de 43% à 93%, de préférence 55% à 70% et de manière préférée 58% à 65% en poids par rapport au poids total de la composition.

4. Composition pharmaceutique selon l'une quelconque des revendications 1 à 3 dans laquelle la phase huileuse vegétale de l'émulsion de Pickering comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/ caprique.

5. Composition pharmaceutique selon l'une quelconque des revendications 1 à 4 dans laquelle la phase huileuse de l'émulsion de Pickering est présente en une quantité allant de 5% à 40%, de préférence 10% à 30% et de manière préférée 15% à 25% en poids par rapport au poids total de la composition.

6. Composition pharmaceutique selon l'une quelconque des revendications 1 à 5 comprenant, dans un milieu acceptable, au moins un agent additionnel choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

7. Composition pharmaceutique selon l'une quelconque des revendications 1 à 6 dans laquelle la composition est appliquée par pulvérisation.

8. Composition pharmaceutique selon l'une quelconque des revendications 1 à 7 pour son utilisation pour augmenter la production de sérotonine.

9. Composition pharmaceutique selon l'une quelconque des revendications 1 à 8 pour son utilisation en tant qu'agent anti inflammatoire cutané.

10. Composition pharmaceutique selon l'une quelconque des revendications 1 à 8 pour son utilisation en tant qu'agent anti-allergie cutanée.

11. Composition pharmaceutique selon la revendication 9 pour son utilisation pour la prévention et/ou le traitement d'une maladie inflammatoire de la peau de préférence choisie dans le groupe formé par l'acné, la rosacée, la folliculite, la dermite périorale, le photovieillissement, le vieillissement cutané, le psoriasis, l'ichtyose, les plaies chroniques, les escarres, la kératose pilaire, les kystes sébacés, les dermatoses inflammatoires, l'hyperpigmentation post-inflammatoire, la xérose, le prurit, le lichen plan, le prurigo nodulaire, l'eczéma, la fièvre miliaire, la sclérodermie, la dermatite atopique, la dermopathie fibrosante néphrogénique, la connectivité mixte, le scléromyxoedème, le scléroedème, la chéloïde, la sclérodactylie, la fasciite à éosinophiles , la photodermatose, les ulcères de stase veineuse ou d'ulcères du pied diabétique, la fibrose cutanée.

Europäisches Patentamt
European Patent Office
Office européen des brevets

# RAPPORT DE RECHERCHE EUROPEENNE

Numéro de la demande
EP 25 21 8373

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2020/064907 A1 (INST NAT SANTE RECH MED [FR]; UNIV AIX MARSEILLE [FR] ET AL.) 2 avril 2020 (2020-04-02) * alinéa [0088]; revendications 1,3,4; tableaux 6,7 * ----- | 1-11 | INV. A61K8/06 A61K8/26 A61K8/92 A61P17/10 A61Q19/00 A61Q19/08 A61K9/00 A61K47/44 |
| X | US 2021/085577 A1 (BOURGETEAU VINCENT [FR] ET AL) 25 mars 2021 (2021-03-25) * revendications 1,3 * ----- | 1-11 | |
| A,D | QIU ET AL.: "TAFA4-IL-10 axis potentiate immunotherapy for airway allergy by induction of specific regulatory T cells", NPJ VACCINES, vol. 7, 2022, page 133, XP002813442, * le document en entier * ----- | 8-11 | |
| A,D | GUILLAUME HOEFFELGUILHAUME DEBROASANAIS ROGERRAFAËLLE ROSSIGNOLJORDI GOUILLY ET AL.: "Sensory neuron-derived TAFA4 promotes macrophage tissue repair functions", NATURE, vol. 594, no. 7861, 2021, pages 94-99, XP037471021, * le document en entier * ----- | 8-11 | **DOMAINES TECHNIQUES RECHERCHES (IPC)** A61K A61P A61Q |
| A,D | ZHU SHU XBENNETT SMAI YXU J: "Molecular Structure, Expression and Role of TAFA4 and its Receptor FPR1 in the Spinal Cord", FRONT IERS IN CELL AND DEV ELOPMENTAL BIOLOGY, 2022, XP093266175, * le document en entier * ----- | 8-11 | |

Le présent rapport a été établi pour toutes les revendications

2

| Lieu de la recherche | Date d'achèvement de la recherche | Examinateur |
|---|---|---|
| Munich | 7 avril 2026 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 25 21 8373

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-04-2026

| Document brevet cité au rapport de recherche | Date de publication | Membre(s) de la famille de brevet(s) | Date de publication |
|---|---|---|---|
| WO 2020064907 A1 | 02-04-2020 | EP 3856228 A1 | 04-08-2021 |
| | | ES 2943136 T3 | 09-06-2023 |
| | | US 2022031807 A1 | 03-02-2022 |
| | | WO 2020064907 A1 | 02-04-2020 |
| US 2021085577 A1 | 25-03-2021 | CA 3002130 A1 | 20-04-2017 |
| | | EP 3362035 A1 | 22-08-2018 |
| | | US 2021085577 A1 | 25-03-2021 |
| | | WO 2017064686 A1 | 20-04-2017 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2020064907 A
- FR 2976503


### Littérature non-brevet citée dans la description


- **ZHU S** ; **HU X** ; **BENNETT S** ; **MAI Y** ; **XU J.** Molecular Structure, Expression and Role of TAFA4 and its Receptor FPR1 in the Spinal Cord.. *Frontiers in Cell and Developmental Biology*, 2022, vol. 10, 911414
- **GUILLAUME HOEFFEL** ; **GUILHAUME DEBROAS** ; **ANAIS ROGER** ; **RAFAËLLE ROSSIGNOL** ; **JORDI GOUILLY et al.** Sensory neuron-derived TAFA4 promotes macrophage tissue repair functions. *Nature*, 2021, vol. 594 (7861), 94-99
- **CHANG** ; **G GU**. Effects on tactile transmission by serotonin transporter inhibitors at Merkel discs of mouse whisker hair follicles. *Molecular Pain*, 20 May 2020, vol. 16, 1-9
- **QIU et al.** TAFA4-IL-10 axis potentiate immunotherapy for airway allergy by induction of specific regulatory T cells. *npj Vaccines*, 2022, vol. 7, 133